# EUROPÄISCHE PATENTSCHRIFT

(11) **EP 0 430 019 B1**
(45) Veröffentlichungstag und Bekanntmachung des Hinweises auf die Patenterteilung: **13.03.1996**
(21) Anmeldenummer: 90122120.0
(22) Anmeldetag: 20.11.1990
(51) Int. Cl.: A61K 9/70, A61L 15/44

(54) **Transdermales therapeutisches System mit Buprenorphin als aktivem Bestandteil**
Transdermal therapeutic system containing buprenorphine as an active component
Système thérapeutique transdermique contenant de la buprénorphine comme composant actif

(30) Priorität: 29.11.1989 DE 3939376
(43) Veröffentlichungstag der Anmeldung: 05.06.1991
(73) Patentinhaber: LTS Lohmann Therapie-Systeme GmbH & Co. KG, D-56513 Neuwied (DE)
(72) Erfinder: Hille, Thomas, Dr., W-5450 Neuwied 1 (DE); Hoffmann, Hans-Rainer, Dr., W-5450 Neuwied 22 (DE); Deurer, Lothar, W-5400 Koblenz 1 (DE)
(74) Vertreter: Flaccus, Rolf-Dieter, Dr.

(56) Entgegenhaltungen:
- EP-A- 0 171 742
- EP-A- 0 242 827
- EP-A- 0 282 156
- EP-A- 0 374 725
- WO-A-88/09676
- US-A- 4 844 903

## Beschreibung

Die Erfindung betrifft ein transdermales therapeutisches System (TTS), das als aktiven Bestandteil Buprenorphin (17-(Cyclopropylmethyl)-α-(1,1-dimethylethyl)-4,5-epoxy-18,19--dihydro-3-hydroxy-6-methoxy-α- methyl-6,14-ethenomorphinan-7-methanol) enthält.

Buprenorphin ist ein partialsynthetisches Opiat, dessen Vorteil gegenüber anderen Verbindungen dieser Substanzklasse in einer höheren Wirksamkeit liegt. Dies bedeutet, daß Schmerzfreiheit bei Krebs- oder Tumorpatienten mit infauster Diagnose im Finalstadium mit Tagesdosen um 1 mg erreicht werden kann. Zwei erhebliche Probleme der Opiate löst Buprenorphin jedoch nicht, nämlich das Suchtpotential und die geringe Bioverfügbarkeit dieser Stoffe bei oraler Gabe. So beträgt die Bioverfügbareit aus dem Gastrointestinaltrakt nur annähernd 10% und bei sublingualer Applikation auch nur etwa 50%.

Die ursprüngliche Annahme eines geringen Suchtpotentials des Buprenorphins nach seiner Einführung als Schmerzmittel wurde korrigiert. Nach Zunahme des Mißbrauchs durch Drogenabhängige unterliegt das Buprenorphin inzwischen dem bundesdeutschen Betäubungsmittelgesetz.

Unter Fachleuten wird allerdings in jüngster Vergangenheit die These vertreten, daß zum Suchtpotential eines Arzneistoffs die Arzneiform beiträgt. Dies ist für stark wirksame Analgetika in der Therapie von extrem hohen Schmerzen leicht nachvollziehbar.

Unmittelbar nach der Applikation ist der Blutspiegel des Schmerzmittels höher als erforderlich und verursacht Euphorien, fällt dann aber exponentiell ab und erreicht rasch Blutspiegel, die den Schmerz nicht mehr erfolgreich therapieren. Aufgrund seiner Schmerzen beginnt der Patient sich nach der nächsten Dosis zu sehnen, wodurch iatrogen eine Sucht erzeugt wird.

Eine Dauerinfusion wäre also bei Buprenorphin und anderen stark wirksamen Opiaten die Arzneiform der Wahl, um diese iatrogene Suchterzeugung durch konstante Blutspiegel zu vermeiden.
Eine Dauerinfusion kann jedoch in der häuslichen Pflege nicht ohne ärztliche Hilfe angelegt und kontrolliert werden und führt oft zu Entzündungen an der Eintrittstelle der Kanüle.

Auch eine orale Depotform kann für Buprenorphin nicht das geeignete Arzneisystem sein, da aufgrund der geringen Bioverfügbarkeit bei oraler Applikation im Vergleich zur erforderlichen intravenösen Dosierung die etwa zehnfache Menge an Wirkstoff abgegeben werden muß. Hier aber bereitet Buprenorphin als partieller Opiat-Antagonist insofern große Probleme, da eine durch Überdosierung des Wirkstoffs hervorgerufene Atemdepression nicht durch die Gabe eines Antagonisten, etwa von Nalorphin, dem Antidot der Wahl bei Opiatvergiftungen, therapierbar ist. Zu Überdosierungen kann es kommen, da die orale Bioverfügbarkeit für Buprenorphin zwar mit 10% angegeben ist, diese aber durchaus höher sein kann, da Buprenorphin einem Patientenkreis gegeben werden soll, bei dem mit Leberfunktionsstörungen zu rechnen ist, so daß durchaus mehr als 10% die erste Leberpassage unmetabolisiert überstehen können.

Darüber hinaus sind orale Depotformen nicht in jeder Hinsicht optimal, wie die Entwicklung der letzten Jahre auf dem Arzneimittelmarkt deutlich zeigt. Generika mit gleicher in-vitro-Freisetzung wie die Präparate der Originalanbieter besitzen nicht die gleiche Wirksamkeit wie eben diese Originalpräparate. Dies bedeutet, daß es durch unkontrollierte Freisetzung in vivo zu Über- oder Unterdosierungen kommen kann. Beides ist bei Buprenorphin besonders fatal. Wird unterdosiert, erleidet der Patient starke Schmerzen. Wird überdosiert, kann es im schlimmsten Fall zu Atemdepressionen mit tödlichem Ausgang kommen, die nicht mit Nalorphin therapierbar sind.
Unberücksichtigt blieb bisher, daß eine orale Depotform, die beschädigt wurde und deshalb Buprenorphin nicht verzögert, sondern auf einen Schlag freisetzt (im Englischen unter Fachleuten als "dosedumping" bekannt), nicht unmittelbar aus dem menschlichen Körper entfernt werden kann.

Alle bisher geschilderten Vorbehalte gegen eine Buprenorphin retardiert freisetzende Arzneiform werden durch die Vorzüge der transdermalen Therapie-Systeme vermieden, da das Medikament nicht über Kanülen in den menschlichen Körper eingebracht werden muß und daher auch von medizinischen Laien appliziert werden kann. Gleichzeitig ist ständig eine Arzneistoffzufuhr nach o.Ordnung sichergestellt, die jederzeit durch Abreißen des Systems unterbrochen werden kann. Ein transdermales therapeutisches System erscheint also für Buprenorphin als Arzneiform der Wahl.

Dem steht jedoch entgegen, daß Buprenorphin durch sein hohes Molekulargewicht (M.G. 468) und vor allem aber durch seinen hohen Schmelzpunkt und seine überaus schlechte Löslichkeit in gängigen organischen Lösungsmitteln und Wasser nur ausgesprochen schlecht durch menschliche Haut penetriert, denn eine Diffusion, die Voraussetzung für die Penetration durch menschliche Haut ist, erfordert gelöste Substanzen. Die Löslichkeit darf aber nicht durch Salzbildung erhöht werden, denn in ionisierter Form werden Basen nicht resorbiert.
Bis heute ist es nicht gelungen, Buprenorphin transdermal in der erforderlichen Menge zur Resorption zu bringen, obwohl aus oben geschilderten Gründen ein TTS für diesen Wirkstoff die bestmögliche Arzneiform darstellt.

So ist in der EP-A-242 827 ein flächenförmiges therapeutisches System mit flexibler Rückschicht, einem oder mehreren Wirkstoffreservoirs, einer oder mehreren haftklebenden Schichten und einer ablösbaren Schutzschicht für die Hautkontaktoberfläche beschrieben. Als Wirkstoff ist unter vielen anderen auch Buprenorphin genannt, auch zur Anwendung in Kombination mit anderen der genannten Wirkstoffe. Es ist nicht angegeben, wie Buprenorphin transdermal in ausreichender Menge zur Resorption gebracht werden kann. Gegenstand der Erfindung ist auch ein Verfahren zur Herstellung des Systems. Dieses Verfahren weist die Schritte auf: Verlegen einer abweisenden Unterlage, Erzeugen einer Hautadhäsionsschicht auf der Unterlage, Zukaschieren eines Stützmaterials, Perforation des derart gebildeten Laminats, Austausch der abweisenden Unterlage gegen eine ablösbare Schutzschicht, Beschichtung der schutzschichtfreien Oberfläche mit wirkstoffhaltiger Reservoirmasse, Zukaschieren der gegebenenfalls haftklebend ausgerüsteten Rückschicht und Konfektionierung. Die Lösung erfordert die Entfernung von Lösungsmittel.

In der EP-A-0 171 742 wird eine pharmazeutische Komposition eines Opioid enthaltenden Wirkstoffs und eines sogenannten "Vehikels" als Übergangsstoff zur transdermalen Lieferung einer therapeutischen Dosis als Wirkstoff beschrieben. Als Arzneiform wird eine Lotion oder Creme definiert. Die Komposition enthält einen Penentrationsbeschleuniger, welcher zumindest aus einem gesättigten Fettalkohol oder einer Fettsäure mit 8 bis 15 Kohlenstoffatomen oder einem ungesättigten Fettalkohol oder einer Fettsäure mit 8 bis 15 Kohlenstoffatomen und einem geeigneten pharmazeutischen Träger, vorzugsweise Propylenglykol, besteht. Eine Angabe, wie eine zufriedenstellende Penetration des als Opioid verwendeten Buprenorphins erhalten wird, ist nicht enthalten.

Schließlich wird noch auf die US-A-4,844,903 hingewiesen, in der ein Pflaster für die trandermale Adsorption von pharmazeutischen Substanzen beschrieben ist. Dort ist u.a. pauschal ausgeführt, daß die transdermale Adsorption in gewünschter Weise durch die Wahl der Konzentration des Wirkstoffs in der Matrix beeinflusst werden kann. Es ist jedoch bekannt, daß diese Maßnahme enge Grenzen hat, insbesondere im Hinblick auf die Löslichkeit eines Wirkstoffs.

Aufgabe der Erfindung ist daher die Bereitstellung von Buprenorphin oder eines seiner pharmazeutisch verträglichen Salze in einem transdermalen therapeutischen System nach dem Oberbegriff von Anspruch 1, das Buprenorphin, oder dessen pharmazeutisch verträgliches Salz über einen Zeitraum von mindestens 24 Stunden kontrolliert abgibt und gewährleistet, daß das Buprenorphin sich während der Lagerung des vorgefertigten transdermalen therapeutischen Systems nicht merklich zersetzt und sicherstellt, daß das unzureichend hautgängige Buprenorphin im geforderten Ausmaß in vivo durch menschliche Haut penetriert, wobei der Aufwand für das System minimiert sein soll.

Diese Aufgabe wird erfindungsgemäß überraschenderweise gelöst durch ein transdermales therapeutisches System in Form eines Pflasters zur Verabreichung von Buprenorphin an die Haut, welches besteht aus einer wirkstoffundurchlässigen Rückschicht, einer haftklebenden Reservoirschicht und einer wiederablösbaren Schutzschicht, und dadurch gekennzeichnet ist, daß die Reservoirschicht 20 bis 90 Gew.-% Polymermaterial, 0,1 bis 30 Gew.-% Weichmacher, 0,1 bis 20 Gew.-% Buprenorphinbase oder eines ihrer pharmazeutisch akzeptablen Salze und 0,1 bis 30 Gew.-% Lösungsmittel für die Wirkstoffbase enthält, und daß das im System verbleibende Lösungsmittel für Buprenorphin in der Reservoirschicht eine Verbindung mit mindestens einer sauren Gruppe ist.

Diese Lösung ist umso erstaunlicher, als Buprenorphin, wie schon erwähnt, sublingual gegeben nur eine Bioverfügbarkeit von 50% aufweist. Da bei dieser Applikationsart die erste Leberpassage umgangen wird, kann die geringe Bioverfügbarkeit nur auf unzureichende Resorbierbarkeit der Substanz durch die Mundschleimhaut zurückgeführt werden. Ein Stoff aber, der die Mucosa des Mundes nur schwer passiert, wird erst recht von der menschlichen Haut nur schwer aufgenommen werden.

Die wirkstoffundurchlässige Rückschicht kann aus flexiblem oder nicht flexiblem Material bestehen. Substanzen, die zu ihrer Herstellung verwendet werden können, sind Polymerfolien oder Metallfolien, wie Aluminiumfolie, die allein oder mit einem polymeren Substrat beschichtet, angewandt werden. Es können auch textile Flächengebilde verwendet werden, wenn die Bestandteile des Reservoirs aufgrund ihrer physikalischen Beschaffenheit durch sie nicht hindurchtreten können. Bei einer bevorzugten Ausführungsform ist die Rückschicht ein Verbundstoff aus einer mit Aluminium bedampften Folie.

Die Reservoirschicht besteht aus einer Polymermatrix und dem Wirkstoff, wobei die Polymermatrix den Zusammenhalt des Systems gewährleistet. Sie besteht aus einem Grundpolymer und gegebenenfalls den üblichen Zusätzen. Die Auswahl des Grundpolymers richtet sich nach den chemischen und physikalischen Eigenschaften des Buprenorphins. Beispielhafte Polymere sind Kautschuk, kautschukähnliche, synthetische Homo-, Co- oder Blockpolymere, Polyacrylsäureester und deren Copolymere, Polyurethane und Silikone. Grundsätzlich kommen alle Polymere in Frage, die bei der Herstellung von Haftklebern eingesetzt werden können und physiologisch unbedenklich sind. Besonders bevorzugt sind solche, die aus Blockcopolymeren auf Basis von Styrol und 1,3-Dienen, Polyisobutylenen, Polymeren auf Acrylat- und/oder Methacrylat-Basis bestehen.

Von den Blockcopolymeren auf Basis von Styrol und 1,3-Dienen werden ganz besonders lineare Styrol-Isopren- oder Styrol-Butadien-Blockcopolymere eingesetzt.

Als Polymere auf Acrylat-Basis werden selbstvernetzende Acrylatcopolymere aus 2-Ethylhexylacrylat, Vinylacetat und Acrylsäure mit bzw. nicht selbstvernetzende Acrylatcopolymere ohne Titanchelatester bevorzugt.

Als Zusätze, die dem Grundpolymer zugesetzt werden, kommen Polymethacrylate, Ester von hydriertem Kolophonium und Polyvinyle in Frage.

Als Methacrylate werden Copolymere auf Basis von Dimethylaminoethylmethacrylaten und neutralen Methacrylsäureestern bevorzugt. Als Ester von hydriertem Kolophonium werden vorzugsweise insbesondere dessen Methyl- und Glycerinester verwendet. Als Polyvinyle werden vorzugsweise Polyvinylpyrrolidone und Polyvinylalkohole eingesetzt.

Die Art der üblichen Zusätze hängt vom eingesetzten Polymer ab: Nach ihrer Funktion lassen sie sich einteilen in beispielsweise Klebrigmacher, Stabilisatoren, Trägerstoffe und Füllstoffe. Die hierfür in Frage kommenden physiologisch unbedenklichen Substanzen sind dem Fachmann bekannt.

Es hat sich erfindungsgemäß gezeigt, daß ein Weichmacher in Verbindung mit einem Lösungsmittel für Buprenorphin erforderlich ist, um Buprenorphin transdermal applizieren zu können.

Die Wahl des Weichmachers richtet sich nach dem Polymer. Besonders geeignet sind höhere Alkohole wie Dodecanol, Undecanol, Octanol, Ester von Carbonsäuren, wobei die Alkoholkomponente auch ein polyethoxylierter Alkohol sein kann, Diester von Dicarbonsäuren, z.B. Di-n-butyladipat sowie Triglyceride, insbesondere mittelkettige Triglyceride der Capryl/Caprinsäuren des Kokosöls. Weitere Beispiele für einen geeigneten Weichmacher sind mehrwertige Alkohole, z.B. Glycerin und Propandiol-(1,2) u.a., die auch durch Polyethylenglykole verethert sein können.

Die Rolle des Lösungsmittels für Buprenorphinbase wird durch die Beispiele belegt. Sie zeigen, daß das Lösungsmittel ein unverzichtbarer Bestandteil der Rezeptur ist. Die Kombination Weichmacher/Lösungsmittel gemäß der Lehre der Erfindung schafft die Voraussetzung für die Penetration der Buprenorphinbase durch die Haut.

Als Lösungsmittel für Buprenorphin in der Matrix kommen solche mit mindestens einer sauren Gruppe in Frage. Besonders geeignet sind die Monoester von Dicarbonsäuren, z.B. Monomethylglutarat und Monomethyladipat. Grundsätzlich kommen alle Säuren in Frage, die Buprenorphin in ausreichendem Maße lösen, ohne daß es zu einer vollständigen Salzbildung kommt, da in letzterem Falle nicht mehr mit einer Penetration durch die Haut gerechnet werden kann.

Die Reservoirschicht besitzt eine solche Eigenklebrigkeit, daß ein dauernder Kontakt zur Haut sichergestellt ist.

Die ablösbare Schutzschicht, die mit der Reservoirschicht in Berührung steht und vor der Anwendung entfernt wird, besteht beispielsweise aus denselben Materialien, wie sie zur Herstellung der Rückschicht benutzt werden, vorausgesetzt, daß sie ablösbar gemacht werden, wie z.B. durch eine Siliconbehandlung. Andere ablösbare Schutzschichten sind z.B. Polytetrafluorethylen, behandeltes Papier, Cellophan, Polyvinylchlorid u.ä. Wird das erfindungsgemäße Laminat vor Aufbringen der Schutzschicht in therapiegerechte Formate (Pflaster) aufgeteilt, so können die dann aufzubringenden Schutzschichtformate ein überstehendes Ende aufweisen, mit dessen Hilfe sie leichter von dem Pflaster abgezogen werden können.

Das erfindungsgemäße transdermale therapeutische System wird hergestellt, indem der Wirkstoff zusammen mit den Bestandteilen der haftklebenden Reservoirschicht gegebenenfalls in Lösung homogen vermischt und auf die wirkstoffundurchlässige Rückschicht aufgestrichen wird, worauf gegebenenfalls das Lösemittel oder die Lösemittel entfernt wird/werden. Anschließend wird die Klebeschicht mit einer entsprechenden Schutzschicht versehen.

Auch der umgekehrte Weg, daß die Kleberlösung auf die Schutzschicht aufgestrichen wird, ist grundsätzlich möglich. Man entfernt auch in diesem Fall die Lösungsmittel und deckt dann anschließend mit der Rückschicht ab.

Die Erfindung wird durch die folgenden Beispiele erläutert:

### Beispiel I:

Je 10,0 g Glutarsäuremonomethylester, Methanol und Butanon und 15,0 g 1-Dodecanol werden unter Rühren gemischt. Anschließend werden 10,0 g Buprenorphinbase eingetragen; man rührt bis zum vollständigen Auflösen des Feststoffs (ca. 30 min, visuelle Kontrolle). Danach werden unter Rühren 133,0 g eines selbstvernetzenden Acrylatcopolymeren aus 2-Ethylhexylacrylat, Vinylacetat und Acrylsäure 46%ig in einem Lösungsmittelgemisch (Ethylacetat: Heptan: Isopropanol: Toluol: Acetylaceton 37 : 26 : 26 : 4 : 1) zugegeben; es wird homogenisiert. Danach werden unter Rühren noch zusätzlich 1,3 g Aluminiumacetylacetonat eingestreut und 3 Stunden lang bei Raumtemperatur gerührt. Der Verdunstungsverlust wird ausgeglichen.
Es resultieren 189,3 g 52,8%ige (G/G) wirkstoffhaltige Kleberlösung, die mit einem 350 µm Rakel auf eine aluminisierte und silikonisierte Polyethylenfolie gestrichen wird. Nachdem die Lösungsmittel durch 30 minütiges Trocknen bis 60 °C entfernt wurden, deckt man den Klebefilm mit einer Polyesterfolie 15 µm ab. Mit geeigneten Schneidewerkzeugen stanzt man eine Fläche von 16 cm² aus und entfernt die Ränder durch Abgittern. Die Freisetzung dieses und der anderen Rezepturbeispiele sind in der Tabelle angegeben; dort sind sowohl die kontrollierte Freisetzung in eine physiologische Kochsalzlösung als auch durch exzidierte Nagetierhaut aufgeführt.

Alle weiteren Beispiele werden nach dem unter Beispiel I angegebenen Schema angefertigt. Zunächst werden immer die flüssigen Bestandteile gemischt, dann Buprenorphinbase eingestreut. Nach dessen Auflösung wird gegebenenfalls ein Methacrylatcopolymer auf Basis von Dimethylaminoethylmethacrylat und neutralen Methacrylsäureestern zugesetzt und nach dessen Auflösung die Kleberlösung zugefügt. In der folgenden Tabelle sind die Rezepturbestandteile nach Trocknen aufgeführt. Dabei bedeuten:
- Acrylat:: Acrylatcopolymeres aus 2-Ethylhexylacrylat, Vinylacetat und Acrylsäure
- Halbester:: Monomethylester der Glutar- (gekennzeichnet durch G) bzw. Adipinsäure (gekennzeichnet durch A)
- G.L.:: polyethoxyliertes Glycerin mit C₈/C₁₀-Ethoxygruppen
- Polymerzusätze:: b: Copolymer mit basischem Charakter auf Basis von Dimethylaminoethylmethacrylat und neutralen Methacrylsäureestern n: Copolymer mit neutralem Charakter auf der Basis von Methacrylsäuremethylester und Methacrylsäurebutylester PVP: Polyvinylpyrrolidon
Die in vitro-Freisetzung wurde in einem Schüttelwasserbad bei 37 °C bestimmt. Das Akzeptormedium waren 100 ml physiologische Kochsalzlösung, die nach 2, 4 und 8 Stunden komplett ausgewechselt wurden. Die Konzentration wurden nach 2, 4 und 8 und 24 Stunden per HPLC bestimmt. Die Penetration an der Mäusehaut wurde an Franz'schen Diffusionszellen gemessen.

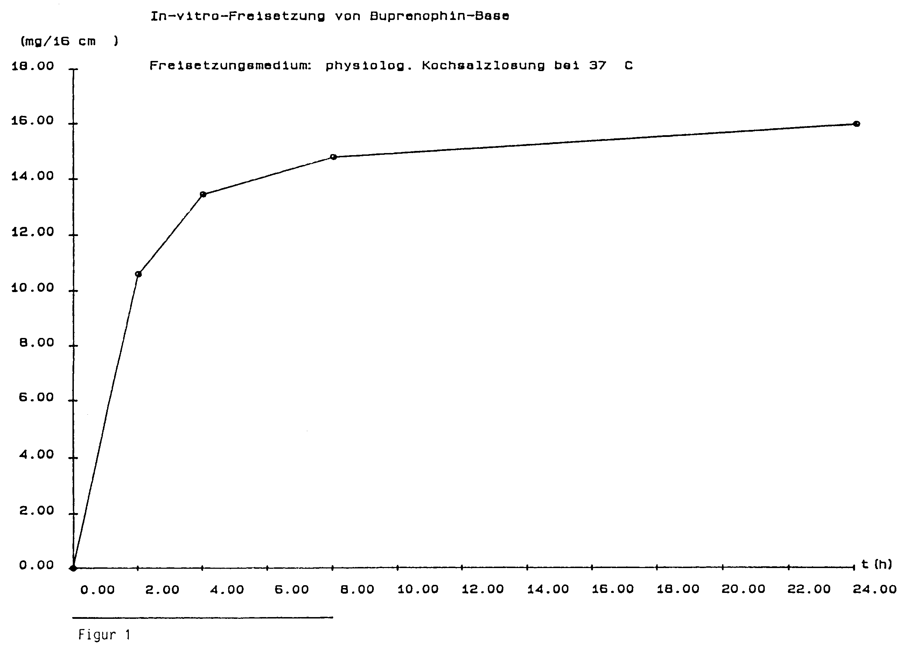

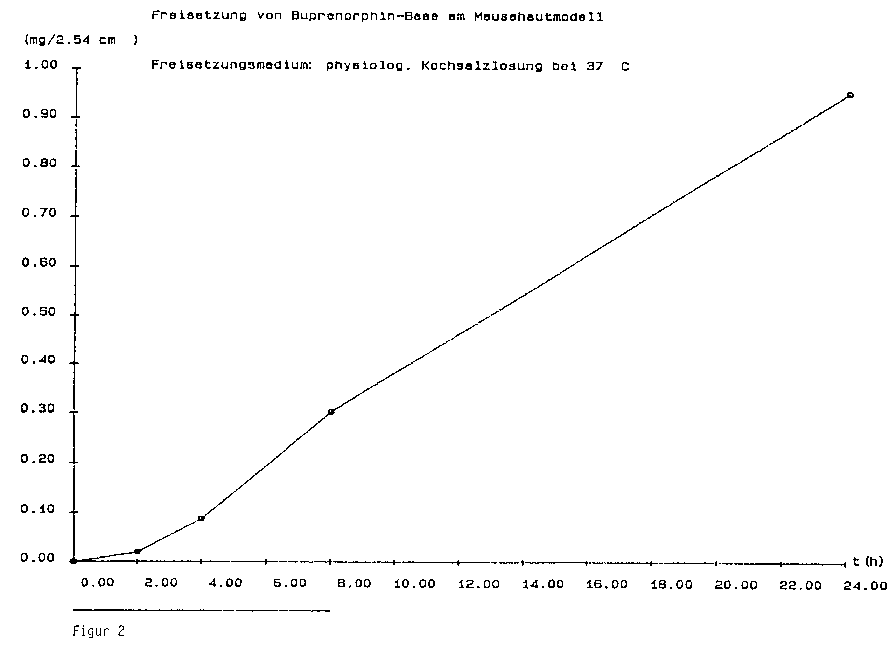

Die Freisetzungskurven gemäß Beispiel I sind in Figur 1 und 2 dargestellt.

| Beispiel | Acrylat | Buprenorphin | Halbester | Weichmacher | | Polymerzusatz | Freisetzung [mg/16 cm³x24h] | Penetration der Mäusehaut [mg/2,54cm²24h] |
|---|---|---|---|---|---|---|---|---|
| I | 65 % | 10 % | 10 % G | 1-Dodecanol | 15 % | - | 16,0 = 74,6 % | 0,95 |
| II | 65 % | 10 % | 10 % G | G.L. | 10 % | 5 % b | 13,6 = 68 % | 0,57 |
| III | 60 % | 10 % | 10 % G | 1-Dodecanol | 10 % | 10 % b | 17,9 = 85 % | 0,47 |
| IV | 60 % | 10 % | 10 % G | G.L. | 10 % | 10 % n | - | 0,92 |
| V | 50 % | 10 % | 10 % G | G.L. | 0 % | 20 % n | - | 0,71 |
| VI | 40 % | 10 % | 10 % G | G.L. | 20 % | 20 % n | - | 0,56 |
| VII | 50 % | 10 % | - | G.L. | 20 % | 20 % n | - | 0,09 |
| VIII | 80 % | 10 % | 5 % G | G.L. | 5 % | - | - | 0,28 |
| IX | 67,5 % | 10 % | 10 % G | G.L. | 10 % | PVP 2,5 % | - | 0,78 |
| X | 65 % | 10 % | 10 % G | G.L. | 10 % | 5 % b | - | 0,44 |
| XI | 65 % | 10 % | 10 % G | 1-Dodecanol | 10 % | 5 % b | 14,6 = 77,3 % | 0,81 |
| XII | 75 % | 10 % | 10 % G | - | | 5 % b | - | 0,22 |
| XIII | 70 % | 10 % | 2,5 % G | 1-Dodecanol | 17,5% | - | - | 0,48 |
| XIV | 80 % | 10 % | - | 1-Dodecanol | 10 % | - | - | 0,11 |
| XV | 72,5 % | 10 % | 2,5 % G | 1-Dodecanol | 10 % | 5 % b | - | 0,51 |
| XVI | 65 % | 10 % | 5 % G | 1-Dodecanol | 15 % | 5 % b | - | 0,4 |
| XVII | 65 % | 10 % | - | 1-Dodecanol | 20 % | 5 % b | - | 0,1 |
| XVIII | 70 % | 10 % | 10 % G | 1-Dodecanol | 10 % | - | 13,6 = 65 % | 0,84 |
| XIX | 60 % | 10 % | 10 % G | 1-Dodecanol | 10 % | 10 % n | 15,3 = 68 % | 0,94 |
| XX | 70 % | 10 % | 5 % G | 1-Dodecanol | 15 % | - | 14,6 = 68,6 % | 0,64 |
| XXI | 65 % | 10 % | 10 % A | 1-Dodecanol | 15 % | - | 16,5 = 73,1 % | 0,85 |

### Interpretation der in vitro-Ergebnisse

Die Beispiel VII, XIV und XVII belegen die Notwendigkeit, einen Lösungsvermittler mit mindestens einer sauren Gruppe in die transdermalen Systeme einzuarbeiten, da ohne ein solches Lösungsmittel die in vitro-Penetration offensichtlich drastisch abnimmt. Die in vitro-Penetration liegt bei allen drei Beispielen bei 0,1 mg/2,54 cm² x h. Gleichzeitig zeigen die Beispiele I und XXI, daß es praktisch für die in vitro-Penetration keine Rolle spielt, ob Glutarsäure- oder Adipinsäuremonomethylester eingesetzt wird. Beispiel XII dient als Beleg dafür, daß neben dem Lösungsvermittler noch ein Weichmacher eingesetzt werden muß, denn ohne einen solchen liegt die in vitro-Penetration mit 0,22 mg/2,54 cm² x 24 h nur wenig über der der Systeme ohne Lösungsvermittler.

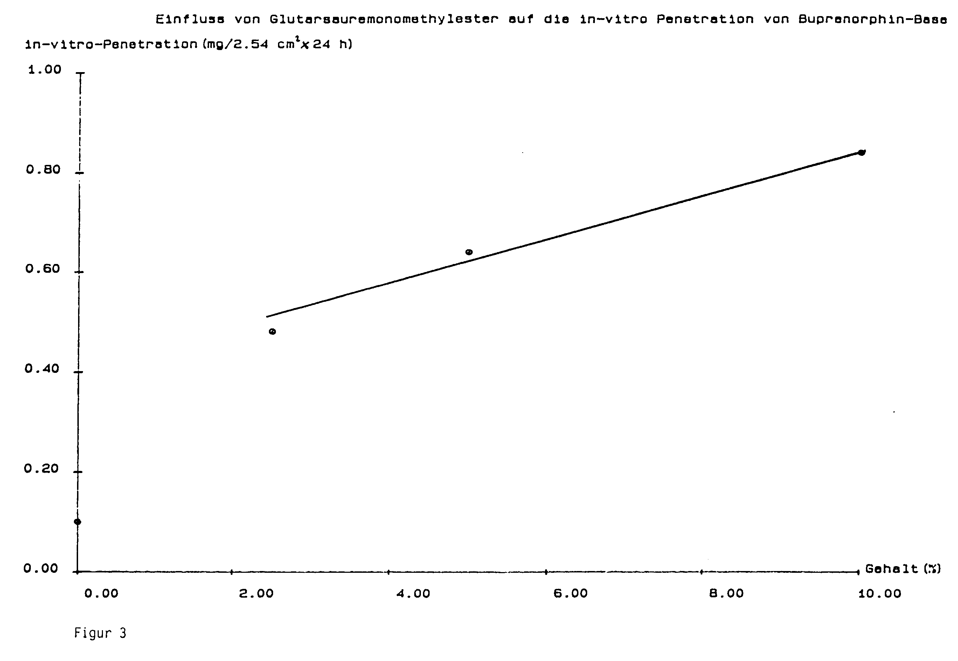

Die Beispiele XIV, XIII, XX und XVIII dienen der Untersuchung des Einflusses der Quantität der Halbester auf die in vitro-Penetration; in dieser Reihefolge wurde der Halbesteranteil von 0% über 2,5% und 5% auf 10% erhöht. Dadurch stieg die in vitro-Penetration an der Mäusehaut von 0,1 über 0,48 und 0,64 auf 0,84 mg/2,54 cm² x 24 h. Nach Zugabe von Halbester ist der Anstieg der in vitro-Penetration annähernd linear. Dies wird in der folgenden Figur 3 wiedergegeben.

Der Vergleich der Beispiele X und XI zeigt, daß 1-Dodecanol als Weichmacher bevorzugt ist. Die übrigen Beispiele zeigen, wie sich Polymerzusätze auf die in vitro-Penetration auswirken, da der Einsatz dieser Stoffe notwendig ist, um Filmbildung, Klebkraft, Adhäsion und Kohäsion zu gewährleisten.

## Patentansprüche (Patentansprüche für folgende(n) Vertragsstaat(en): AT, BE, CH, DE, DK, FR, GB, IT, LI, LU, NL, SE)

1. Transdermales therapeutisches System in Form eines Pflasters zur Verabreichung von Buprenorphin an die Haut aus einer wirkstoffundurchlässigen Rückschicht, einer haftklebenden Reservoirschicht und einer wiederablösbaren Schutzschicht, dadurch gekennzeichnet, daß die Reservoirschicht 20-90 Gew.-% Polymermaterial, 0,1-30 Gew.-% Weichmacher, 0,1-20 Gew.-% Buprenorphinbase oder eines ihrer pharmazeutisch akzeptablen Salze und 0,1-30 Gew.-% Lösungsmittel für die Wirkstoffbase enthält, und daß das im System verbleibende Lösungsmittel für Buprenorphin in der Reservoirschicht eine Verbindung mit mindestens einer sauren Gruppe ist.

2. Transdermales therapeutisches System nach Anspruch 1, dadurch gekennzeichnet, daß die Verbindung mit mindestens einer sauren Gruppe ein Monoester einer Dicarbonsäure ist.

3. Transdermales therapeutisches System nach Anspruch 2, dadurch gekennzeichnet, daß der Monoester einer Dicarbonsäure Glutarsäure- oder Adipinsäuremonomethylester ist.

4. Transdermales therapeutisches System nach Anspruch 1, dadurch gekennzeichnet, daß das Polymermaterial Polymere auf Basis von Methacrylaten enthält, vorzugsweise ein Copolymer auf Basis von Dimethylaminoethylmethacrylat und neutralen Methacrylsäureestern oder auf Basis von Methacrylsäuremethylester und Methacrylsäurebutylester.

5. Transdermales therapeutisches System nach Anspruch 1, dadurch gekennzeichnet, daß das Polymermaterial Zusätze auf Basis von hydriertem Kolophonium, vorzugsweise seines Methyl- oder Glycerinesters enthält.

6. Transdermales therapeutisches System nach Anspruch 1, dadurch gekennzeichnet, daß das Polymermaterial Polyvinylpyrrolidon oder Polyvinylalkohol enthält.

7. Transdermales therapeutisches System nach Anspruch 1, dadurch gekennzeichnet, daß die Reservoirschicht als Weichmacher Dodecanol enthält.

8. Transdermales therapeutisches System nach Anspruch 1, dadurch gekennzeichnet, daß die Reservoirschicht als Weichmacher polyethoxyliertes Glycerin mit C₈/C₁₀-Ethoxygruppen, deren freie Hydroxylgruppen teilweise mit Capryl/Caprinsäuren verestert sind, enthält.

9. Transdermales therapeutisches System nach Anspruch 1, dadurch gekennzeichnet, daß das Polymermaterial lineares Styrol-Butadien-Styrol- oder Styrol-Isopren-Styrol-Blockcopolymeres enthält.

10. Transdermales therapeutisches System nach Anspruch 1, dadurch gekennzeichnet, daß das Polymermaterial selbstvernetzendes Acrylatcopolymeres aus 2-Ethylhexylacrylat, Vinylacetat, Acrylsäure und Titanchelatester oder nicht selbst vernetzendes Acrylatcopolymeres aus 2-Ethylhexylacrylat, Vinylacetat und Acrylsäure enthält.

11. Transdermales therapeutisches System nach Anspruch 1, dadurch gekennzeichnet, daß die Rückschicht aus flexiblem oder inflexiblem Material, vorzugsweise einem Verbundstoff aus einer mit Aluminium bedampften Folie, aufgebaut ist.

## Patentansprüche (Patentansprüche für folgende(n) Vertragsstaat(en): ES, GR)

1. Verfahren zur Herstellung eines transdermalen therapeutischen Systems zur Verabreichung von Buprenorphin an die Haut aus einer wirkstoffundurchlässigen Rückschicht, einer haftklebenden Reservoirschicht und einer wiederablösbaren Schutzschicht, dadurch gekennzeichnet, daß zur Herstellung der Reservoirschicht ein Gemisch aus 20 bis 90 Gew.-% Polymermaterial, 0,1 bis 30 Gew.-% Weichmacher, 0,1 bis 20 Gew.-% Buprenorphinbase oder einen ihrer pharmazeutisch akzeptablen Salze und 0,1 bis 30 Gew.-% Lösungsmittel für die Wirkstoffbase unter eventuellem Zusatz leicht flüchtiger Lösungsmittel homogenisiert und auf die Rückschicht oder Schutzschicht aufgetragen wird, wonach leicht flüchtige Lösungsmittel entfernt werden.

2. Verfahren nach Anspruch 1, dadurch gekennzeichnet, daß zur Herstellung der Reservoirschicht, die 20 bis 90 Gew.-% Polymermaterial, 0,1 bis 30 Gew.-% Weichmacher, 0,1 bis 20 Gew.-% Buprenorphinbase oder eines ihrer pharmazeutisch akzeptablen Salze und 0,1 bis 30 Gew.-% Lösungsmittel für die Wirkstoffbase enthält, zunächst die flüssigen Lösungsmittel- und Weichmacherkomponenten gemischt, dann der Buprenorphinwirkstoff eingetragen und nach dessen Auflösung eine das Polymermaterial enthaltende Kleberlösung zugegeben wird,
und daß nach Homogenisierung die wirkstoffhaltige Kleberlösung auf die Rück- bzw. Schutzschicht aufgebracht und das leicht flüchtige Lösungsmittel entfernt wird.

3. Verfahren nach Anspruch 1, dadurch gekennzeichnet, daß die wirkstoffhaltige Kleberlösung auf eine Rückschicht aus flexiblem oder inflexiblem Material, vorzugsweise einem Verbundstoff aus einer mit Aluminium bedampften Folie, aufgetragen wird.

4. Verfahren nach Anspruch 1, dadurch gekennzeichnet, daß die Reservoirschicht unter Verwendung eines Polymermaterials aus linearem Styrol-Butadien-Styrol- oder Styrol-Isopren-Styrol-Blockpolymerem hergestellt wird.

5. Verfahren nach Anspruch 1, dadurch gekennzeichnet, daß die Reservoirschicht unter Verwendung eines Polymermaterials aus selbstvernetzendem Acrylcopolymeren aus 2-Ethylhexylacrylat, Vinylacetat, Acrylsäure und Titanchelatester oder nicht vernetzenden Acrylatcopolymerem aus 2-Ethylhexylacrylat, Vinylacetat und Acrylsäure hergestellt wird.

6. Verfahren nach Anspruch 1, dadurch gekennzeichnet, daß die Reservoirschicht unter Verwendung eines Polymermaterials hergestellt wird, welchem Polymere auf Basis von Methacrylaten zugesetzt werden, vorzugsweise ein Copolymer auf Basis von Dimethylaminoethylmethacrylat und neutralen Methacrylsäureestern oder auf Basis von Methacrylsäuremethylester und Methacrylsäurebutylester.

7. Verfahren nach Anspruch 1, dadurch gekennzeichnet, daß die Reservoirschicht unter Verwendung eines Polymermaterials hergestellt wird, dem Zusätze auf Basis von hydriertem Kollophonium, vorzugsweise seines Methyl- oder Glycerinesters, zugesetzt werden.

8. Verfahren nach Anspruch 1, dadurch gekennzeichnet, daß die Reservoirschicht unter Verwendung eines Polymermaterials hergestellt wird, dem Polyvinylpyrrolidon oder Polyvinylalkohol zugesetzt werden.

9. Verfahren nach Anspruch 1, dadurch gekennzeichnet, daß die Reservoirschicht unter Verwendung von Dodecanol als Weichmacher hergestellt wird.

10. Verfahren nach Anspruch 1, dadurch gekennzeichnet, daß die Reservoirschicht unter Verwendung von polyethoxyliertem Glycerin mit C₈/C₁₀-Ethoxygruppen, deren freie Hydroxylgruppen teilweise mit Capryl/Caprinsäuren verestert sind, als Weichmacher hergestellt wird.

11. Verfahren nach Anspruch 1, dadurch gekennzeichnet, daß als Lösungsmittel für Buprenorphin in der Reservoirschicht eine Verbindung mit mindestens einer sauren Gruppe eingesetzt wird, vorzugsweise ein Monoester einer Dicarbonsäure, insbesondere bevorzugt Glutarsäure- oder Adipinsäuremonomethylester.

## Claims (Claims for the following Contracting State(s): AT, BE, CH, DE, DK, FR, GB, IT, LI, LU, NL, SE)

1. Transdermal therapeutic system in the form of a plaster, for the administration of buprenorphine to the skin, comprising a backing layer which is impermeable to active substance, a pressure-sensitive adhesive reservoir layer and, optionally, a removable protective layer, characterized in that the reservoir layer comprises 20 to 90%-wt polymeric material, 0.1 to 30%-wt softener, 0.1 to 20%-wt buprenorphine base or one of the pharmaceutically acceptable salts thereof, and 0.1 to 30%-wt solvent for the active substance base, and in that the solvent for buprenorphine remaining in the system in the reservoir layer is a compound having at least one acidic group.

2. The transdermal therapeutic system according to claim 1 wherein the compound with at least one acidic group is a monoester of a dicarboxylic acid.

3. The transdermal therapeutic system according to claim 2 wherein the monoester of a dicarboxylic acid is glutaric acid- or adipic acid-monomethylester.

4. The transdermal therapeutic system according to claim 1 wherein the polymeric material comprises polymers based on methacrylates, preferably a copolymer based on dimethylaminoethyl methacrylate and neutral methacrylic esters or based on methacrylic methylesters and methacrylic butyl esters.

5. The transdermal therapeutic system according to claim 1 wherein the polymeric material comprises additives based on hydrogenated colophony, preferably on the methylesters or glycerol esters of hydrogenated colophony.

6. The transdermal therapeutic system according to claim 1 wherein the polymeric material comprises polyvinylpyrrolidone or polyvinyl alcohol.

7. The transdermal therapeutic system according to claim 1 wherein the reservoir layer comprises dodecanol as softening agent.

8. The transdermal therapeutic system according to claim 1 wherein the reservoir layer comprises as softener polyethoxylated glycerol with C₈/C₁₀-ethoxy groups, part of the free hydroxyl groups of which are esterified with caprylic/capric acids.

9. The transdermal therapeutic system according to claim 1 wherein the polymeric material comprises linear styrene-butadiene-styrene- or styrene-isoprene-styrene blockcopolymer.

10. The transdermal therapeutic system according to claim 1 wherein the polymeric material comprises a self-cross-linking acrylate copolymer of 2-ethyl hexyl acrylate, vinyl acetate, acrylic acid, and titanium chelate ester, or a non-self-cross-linking acrylate copolymer of 2-ethyl hexyl acrylate, vinyl acetate, and acrylic acid.

11. The transdermal therapeutic system according to claim 1 wherein the backing layer is composed of flexible or inflexible material, preferably of a composite of an aluminized film or sheet.

## Claims (Claims for the following Contracting State(s): ES, GR)

1. Process for the production of a transdermal therapeutic system for the administration of buprenorphine to the skin, comprising a backing layer which is impermeable to active substance, a pressure-sensitive adhesive reservoir layer and a removable protective layer, characterized in that for producing the reservoir layer a mixture of 20 to 90%-wt polymeric material, 0.1 to 30%-wt softener, 0.1 to 20%-wt buprenorphine base or one of the pharmaceutically acceptable salts thereof, and 0.1 to 30%-wt solvent for the active substance base, is homogenised, optionally adding readily volatile solvents, and is applied to the backing layer or the protective layer, whereafter any readily volatile solvents are removed.

2. The process according to claim 1, characterized in that for producing the reservoir layer, which comprises 20 to 90%-wt polymeric material, 0.1 to 30%-wt softener, 0.1 to 20%-wt buprenorphine base or one of the pharmaceutically acceptable salts thereof, and 0.1 to 30%-wt solvent for the active substance base, first the liquid solvent components and softener components are mixed, then the active substance buprenorphine is introduced and after its dissolution a solution of adhesive containing the polymeric material is added,
and that after homogenization the active substance-containing solution of adhesive is applied to the backing layer or protective layer and the readily volatile solvent is removed.

3. The process according to claim 1, characterized in that the active substance-containing solution of adhesive is applied to a backing layer of flexible or inflexible material, preferably a composite of an aluminized film or sheet.

4. The process according to claim 1, characterized in that the reservoir layer is produced employing a polymeric material of linear styrene-butadiene-styrene blockcopolymer or styrene-isoprene-styrene blockcopolymer.

5. The process according to claim 1, characterized in that the reservoir layer is produced employing polymeric materials of self-crosslinking acrylic copolymers of 2-ethyl hexyl acrylate, vinyl acetate, acrylic acid and titanium chelate ester, or a non-self-crosslinking acrylate copolymer of 2-ethyl hexyl acrylate, vinyl acetate and acrylic acid.

6. The process according to claim 1, characterized in that the reservoir layer is produced employing a polymeric material to which polymers based on methacrylates are added, preferably a copolymer based on dimethylaminoethyl methacrylate and neutral methacrylic esters or based on methacrylic methyl esters and methacrylic butyl esters.

7. The process according to claim 1, characterized in that the reservoir layer is produced employing a polymeric material to which additives based on hydrogenated colophony, preferably on the methyl or glycerol esters thereof, are added.

8. The process according to claim 1, characterized in that the reservoir layer is produced employing a polymeric material to which polyvinyl pyrrolidone or polyvinyl alcohol are added.

9. The process according to claim 1, characterized in that the reservoir layer is produced employing dodecanol as softener.

10. The process according to claim 1, characterized in that the reservoir layer is produced employing as softener polyethoxylated glycerol with C₈/C₁₀-ethoxy groups, part of the free hydroxyl groups of which are esterified with caprylic/capric acids.

11. The process according to claim 1, characterized in that as solvent for buprenorphine in the reservoir layer a compound having at least one acidic group is used, preferably a monoester of a dicarboxylic acid, particularly preferred glutaric acid monomethyl ester or adipic acid monomethyl ester.

## Revendications (Revendications pour l'(les) Etat(s) contractant(s) suivant(s): AT, BE, CH, DE, DK, FR, GB, IT, LI, LU, NL, SE)

1. Système thérapeutique transdermal sous la forme d'un emplâtre ou timbre destiné à l'administration de la buprénorphine à la peau à partir d'une couche dorsale imperméable au principe actif, d'une couche réservoir autocollante et d'une couche protectrice amovible, caractérisé en ce que la couche réservoir contient 20 à 90% en poids de matière polymérique, 0,1 à 30% en poids de plastifiant, 0,1 à 20% en poids de buprénorphine base ou d'un de ses sels pharmaceutiquement acceptables et 0,1 à 30% en poids d'un solvant pour le principe actif de base et en ce que le solvant subsistant dans le système pour la buprénorphine dans la couche réservoir est un composé comportant au moins un groupe acide.

2. Système thérapeutique transdermique suivant la revendication 1, caractérisé en ce que le composé comportant au moins un groupe acide est un monoester d'un acide dicarboxylique.

3. Système thérapeutique transdermique suivant la revendication 2, caractérisé en ce que le monoester d'un acide dicarboxylique est le glutarate ou l'adipate de monométhyle.

4. Système thérapeutique transdermique suivant la revendication 1, caractérisé en ce que la matière polymérique contient des polymères à base de méthacrylates, de préférence, un copolymère à base de méthacrylate de diméthylaminoéthyle et d'esters de l'acide méthacrylique neutres, ou à base de méthacrylate de méthyle et de méthacrylate de butyle.

5. Système thérapeutique transdermique suivant la revendication 1, caractérisé en ce que la matière polymérique contient des additifs à base de colophane hydratée, de préférence son ester méthylique ou glycérinique.

6. Système thérapeutique transdermique suivant la revendication 1, caractérisé en ce que la matière polymérique contient de la polyvinylpyrrolidone ou du poly(alcool vinylique).

7. Système thérapeutique transdermique suivant la revendication 1, caractérisé en ce que la couche réservoir contient du dodécanol à titre de plastifiant.

8. Système thérapeutique transdermique suivant la revendication 1, caractérisé en ce que la couche réservoir contient, à titre de plastifiant, de la glycérine polyéthoxylée avec des radicaux éthoxy en C₈/C₁₀, dont les groupes hydroxyle libres sont partiellement estérifiés avec des acides caprylique/caproïque.

9. Système thérapeutique transdermique suivant la revendication 1, caractérisé en ce que la matière polymérique contient un copolymère séquencé de styrène-butadiène-styrène, ou de styrène-isoprène-styrène, linéaire.

10. Système thérapeutique transdermique suivant la revendication 1, caractérisé en ce que la matière polymérique contient un copolymère d'acrylate autoréticulant constitué d'acrylate de 2-éthylhexyle, d'acétate de vinyle, d'acide acrylique et d'un ester chélate de titane, ou un copolymère d'acrylate non autoréticulant constitué d'acrylate de 2-éthylhexyle, d'acétate de vinyle et d'acide acrylique.

11. Système thérapeutique transdermique suivant la revendication 1, caractérisé en ce que la couche dorsale est formée d'une matière qui est souple ou qui n'est pas souple, de préférence, d'un lamifié constitué d'une feuille revêtue d'aluminium par dépôt sous vide.

## Revendications (Revendications pour l'(les) Etat(s) contractant(s) suivant(s): ES, GR)

1. Système thérapeutique transdermal sous la forme d'un emplâtre ou timbre destiné à l'administration de la buprénorphine à la peau à partir d'une couche dorsale imperméable au principe actif, d'une couche réservoir autocollante et d'une couche protectrice amovible, caractérisé en ce que, pour la réalisation de la couche réservoir on homogénéise un mélange constitué de 20 à 90% en poids d'une matière polymérique, 0,1 à 30% en poids d'un plastifiant, 0,1 à 20% en poids de buprénorphine base ou d'un de ses sels pharmaceutiquement acceptables et 0,1 à 30% en poids de solvant pour le principe actif de base, sous addition éventuelle d'un solvant aisément volatil et on l'applique sur la couche dorsale ou la couche de protection, puis on élimine le solvant aisément volatil.

2. Procédé suivant la revendication 1, caractérisé en ce que pour la réalisation de la couche réservoir qui contient 20 à 90% en poids de matière polymérique, 0,1 à 30 en poids de plastifiant, 0,1 à 20% en poids de buprénorphine base ou d'un de ses sels pharmaceutiquement acceptables et 0,1 à 30% en poids de solvant pour le principe actif de base, on mélange d'abord le composant plastifiant et le solvant liquides, puis on introduit le principe actif formé du buprénorphine et, après la dissolution de ce principe actif, on ajoute une solution de colle contenant la matière polymérique et en ce que, après homogénéisation, on applique la solution de colle contenant le principe actif sur la couche dorsale ou la couche de protection et on élimine le solvant aisément volatil.

3. Procédé suivant la revendication 1, caractérisé en ce que l'on applique la solution de colle contenant le principe actif sur une couche dorsale constituée d'une matière qui est souple ou qui n'est pas souple, de préférence, un lamifié formé d'une feuille revêtue d'aluminium par dépôt sous vide.

4. Procédé suivant la revendication 1, caractérisé en ce que l'on réalise la couche réservoir en recourant à l'emploi d'une matière polymérique constituée d'un copolymère séquencé de styrène-butadiène-styrène, ou de styrène-isoprène-styrène, linéaire.

5. Procédé suivant la revendication 1, caractérisé en ce que l'on réalise la couche réservoir en recourant à l'emploi d'une matière polymérique constituée de copolymères acryliques autoréticulants formés d'acrylate de 2-éthylhexyle, d'acétate de vinyle, d'acide acrylique et d'esters du type chélate de titane, ou de copolymères d'acrylate non autoréticulants formés d'acrylate de 2-éthylhexyle, d'acétate de vinyle et d'acide acrylique.

6. Procédé suivant la revendication 1, caractérisé en ce que l'on réalise la couche réservoir en recourant à l'emploi d'une matière polymérique à laquelle on ajoute des polymères à base de méthacrylates, de préférence un copolymère à base de méthacrylate de diméthylaminoéthyle et d'esters de l'acide méthacrylique neutres, ou à base de méthacrylate de méthyle et de méthacrylate de butyle.

7. Procédé suivant la revendication 1, caractérisé en ce que l'on réalise la couche réservoir en recourant à l'emploi d'une matière polymérique, à laquelle on ajoute des additifs à base de colophane hydratée, de préférence, son ester méthylique ou glycérinique.

8. Procédé suivant la revendication 1, caractérisé en ce que l'on réalise la couche réservoir en recourant à l'emploi d'une matière polymérique à laquelle on ajoute de la polyvinylpyrrolidone ou du poly(alcool vinylique).

9. Procédé suivant la revendication 1, caractérisé en ce que l'on réalise la couche réservoir en recourant à l'emploi de dodécanol comme plastifiant.

10. Procédé suivant la revendication 1, caractérisé en ce que l'on réalise la couche réservoir en recourant à l'emploi de glycérine polyéthoxylée avec des radicaux éthoxy en C₈/C₁₀, dont les radicaux hydroxyle libres sont partiellement estérifiés avec les acides caprylique/caproïque, comme plastifiant.

11. Procédé suivant la revendication 1, caractérisé en ce que, à titre de solvant pour la buprénorphine dans la couche réservoir, on utilise un composé comportant au moins un groupe acide, de préférence, un monoester d'un acide dicarboxylique, de manière tout particulièrement avantageuse, le glutarate ou l'adipate de monométhyle.
